# EUROPÄISCHE PATENTSCHRIFT

(11) **EP 1 016 644 B1**
(45) Veröffentlichungstag und Bekanntmachung des Hinweises auf die Patenterteilung: **22.01.2003**
(21) Anmeldenummer: 99202578.3
(22) Anmeldetag: 30.07.1999
(51) Int. Cl.: C07C 45/53, C07C 37/08, C07C 49/08, C07C 39/04

(54) **Verfahren zur Herstellung von Phenol und Aceton durch säurekatalysierte Spaltung von Cumolhydroperoxid**
Process for the preparation of phenol and acetone by acid catalyzed cleavage of cumene hydroperoxide
Procédé pour la préparation de phenol et d' acétone par décomposition catalysée par un acide de l' hydroperoxyde de cumène

(30) Priorität: 18.12.1998 DE 19858770
(43) Veröffentlichungstag der Anmeldung: 05.07.2000
(73) Patentinhaber: Phenolchemie GmbH & Co. KG, 45966 Gladbeck (DE)
(72) Erfinder: Weber, Manfred, Dr., 45721 Haltern (DE); Gerlich, Otto, Dr., 45966 Gladbeck (DE); Kleine-Boymann, Michael, Dr., 46244 Bottrop (DE); Pompetzki, Werner, Dr., 46284 Dorsten (DE); Sigg, Reinhard, Dr., 45772 Marl (DE); Michalik, Christian, 45355 Essen (DE); Volke, Jürgen, 45966 Gladbeck (DE)
(74) Vertreter: Sternagel, Fleischer, Godemeyer & Partner

(56) Entgegenhaltungen:
- US-A- 5 254 751
- CHEMICAL ABSTRACTS, vol. 102, no. 22, 3. Juni 1985 (1985-06-03) Columbus, Ohio, US; abstract no. 187061, VASIL'EV V F ET AL: "Phenol and acetone" XP002130781 & SU 1 131 865 A (USSR)

## Beschreibung

Die Erfindung betrifft ein verbessertes Verfahren zur selektiven Spaltung von Cumolhydroperoxid (CHP) in Phenol und Aceton unter Einwirkung einer Säure.

Phenol wird weltweit zum größten Teil aus Cumol gewonnen. Dabei wird Cumol zunächst zu Cumolhydroperoxid (kurz: CHP) oxidiert, wobei man aus Selektivitätsgründen in der Regel Umsätze an Cumol von 20 bis 30 Gew.-% fährt. In einer anschließenden sogenannten Konzentrierung wird der CHP-Gehalt im Oxidat üblicherweise auf 65 bis 90 Gew.-% aufkonzentriert. Es entsteht das sogenannte technische CHP. In der nachfolgenden Spaltung entsteht aus dem technischen CHP unter Einwirkung einer Säure, zumeist Schwefelsäure, Phenol und Aceton. Nach Neutralisation des Spaltproduktes werden die Produkte im sogenannten Aufarbeitungsteil einer Phenolanlage im wesentlichen destillativ aus dem Spaltprodukt gewonnen.

Sowohl die Oxidations- als auch die Spaltreaktion werden von einer unerwünschten Nebenproduktbildung begleitet. Der Spaltung kommt für die Selektivität und die Ausbeute des Gesamtverfahrens eine besondere Bedeutung zu. Parallel zur Spaltung von CHP in Phenol und Aceton wird das zuvor in der Oxidation gebildete Dimethylphenylcarbinol (kurz. DMPC) zu Alphamethylstyrol (kurz: AMS) dehydratisiert. AMS kann im Aufarbeitungsteil zu Cumol hydriert und somit zur Oxidationsstufe wieder zurückgeführt werden. In der CHP-Spaltung finden aber Polymerisationsreaktionen des AMS oder Additionsreaktionen zwischen AMS und Phenol statt, die zur Bildung von Hochsiedern (PolyAMS und Cumylphenole) führen, wodurch die Selektivität und Ausbeute des Gesamtprozesses maßgeblich negativ beeinflußt werden. Die Spaltung muß deshalb technisch so durchgerührt werden, daß die Bildung der genannten Hochsieder weitestgehend unterdruckt wird.

Die technische Durchführung der Spaltung wurde bereits mehrfach beschrieben. In der Regel wird die Spaltung in einem ideal durchmischten Apparat durchgeführt. Die bei der stark exothermen CHP-Spaltung freigesetzte Wärme wird entweder durch Verdampfung von Aceton (Siedekühlung, vgl. US-Patent 5 463 136) oder durch außenliegende Kühler, wie z. B. in US-Patent 4 358 618 beschrieben, abgeführt. In US-Patent 4 358 618 wird beschrieben, wie die Selektivität einer solchen Spaltung durch nachgeschaltete Rohrreaktoren verbessert werden kann. Die Fahrweise ist dabei folgendermaßen: In einem ideal durchmischten Hauptreaktor wird technisches CHP durch Zugabe einer Säure bei Temperaturen im Bereich von 50 bis 90 °C bis auf Restkonzentrationen an CHP von 0,5 bis 5 Gew.-% gespalten. Unter diesen Bedingungen setzen sich wenigstens 40 Gew.-% des enthaltenen DMPC mit CHP zu Dicumylperoxid (kurz: DCP) und Wasser um. Im nachgeschalteten ersten Rohrreaktor wird das CHP auf Restgehalte unter 0,4 Gew.-% gespalten, die Temperaturen sind dabei ähnlich hoch wie im Hauptreaktor. Im zweiten Rohrreaktor wird schließlich das zuvor gebildete DCP in AMS, Phenol und Aceton gespalten, wobei Temperaturen im Bereich von 120 bis 150 °C eingestellt werden. Demnach können der Hauptreaktor und der erste Rohrreaktor als "CHP-Spaltung" zusammengefaßt und der zweite Rohrreaktor als "DCP-Spaltung" bezeichnet werden. Die Idee, das Spaltprodukt nach der eigentlichen CHP-Spaltung nochmal wie in US-Patent 4 358 618 in einem weiteren Rohrreaktor thermisch nachzubehandeln, ist lange bekannt und wurde bereits in US-Patent 2 757 209 beschrieben, wobei für die DCP-Spaltung Temperaturen über 100 °C, bevorzugt 110 bis 120 °C, angegeben werden. Ziel dieser thermischen Nachbehandlung war damals die vollständige Dehydratisierung des DMPC zu AMS. Zusätzlich zu den bereits in der CHP-Spaltung gebildeten Hochsiedern werden bei der DCP-Spaltung weitere Hochsieder gebildet.

Auch das Patentdokument SU 11 31 865 A beschreibt bereits ein zweistufiges Verfahren, in dessen erster Stufe die eigentliche CHP-Spaltung erfolgt, während in der zweiten Stufe in der ersten Stufe gebildetes DCP gespalten wird. SU 11 31 865 A lehrt darüber hinaus zur Warmeabfuhr bezogen auf jede Stufe eine Ruckführung von Produkt der jeweiligen Stufe zum Zulauf der jeweiligen Stufe im Mengenverhältnis von 20.1 sowie die Zuführ der als Katalysator verwendeten Schwefelsäure erst zur zweiten Stufe, so daß eine weitere Rückführung von Spaltprodukt der DCP-Spaltung (zweite Stufe) zum Zulauf der CHP-Spaltung (erste Stufe) im Volumenverhältnis von 1 : 1 bis 1 : 10 erforderlich ist, um die Säure in die erste Stufe zu überführen.

Eigene Untersuchungen haben gezeigt, daß die Spaltungsgeschwindigkeit von Cumolhydroperoxid d CHP/dt proportional zur CHP-Konzentration ist. Somit ist für einen vorgegebenen Umsatz die Raum-Zeit-Ausbeute in einem wie in US-Patent 4 358 618 beschriebenen ideal durchmischten Reaktor immer niedriger als in einem Rohrreaktor. Das Volumen eines solchen ideal durchmischten Apparates ist deshalb immer größer als das eines Rohrreaktors, wenn jeweils gleiche CHP-Mengenströme auf jeweils gleiche CHP-Endgehalte gespalten werden sollen. Entsprechend größer sind die Mengen an Spaltprodukt mit Restgehalten an CHP, die sich in einem ideal durchmischten Apparat im Vergleich zu einem Rohrreaktor befinden. Aus sicherheitstechnischen Gründen ist es aber vorteilhaft, das Volumen in Spaltreaktoren so gering wie möglich zu halten und gleichzeitig eine hohe Wärmeübertragungsfläche zwischen reagierendem technischen CHP und Kühlmedium zu realisieren. Dies wird erreicht, wenn die Spaltung in Reaktoren mit Rohrströmungscharakteristik, also z. B. Rohrbündelwärmeübertragern, durchführt wird. Dabei kann das Produkt sowohl auf der Rohrseite als auch im Mantelraum strömen, wenn hier durch spezielle Einbauten (Umlenkbleche) eine Rohrströmungscharakteristik eingestellt wird. Kleine Reaktionsvolumina und große Warmeubertragungsflachen, d. h. Reaktoren mit großen volumenspezifischen Oberflächen zur Wärmeübertragung, gewährleisten, daß selbst bei Ausfall von z. B. Pumpen trotz der stark exothermen Spaltreaktion von CHP keine sicherheitstechnisch kritischen Zustände auftreten.

Die Verwendung von mehreren hintereinandergeschalteten Rohrbündelwärmeübertragern für die Spaltung von technischem CHP wird bereits in DBP 1 112 527 beschrieben. Dabei wird CHP in überschüssiger Schwefelsäure dispergiert und die Dispersion dann durch die Kühler geleitet, in denen die Reaktionswärme abgeführt wird; anschließend werden Schwefelsäure und organische Phase voneinander getrennt. Die Schwefelsäure wird zurückgeführt, das Spaltprodukt wird neutralisiert und aufgearbeitet. Mit dieser sogenannten heterogenen Spaltung sind aber keine hohen Selektivitäten erreichbar, da in der im Kreis geführten Schwefelsäure verstärkt Nebenreaktionen unter Bildung von Hochsiedern ablaufen.

Ebenso wie in US-Patent 4 358 618 wird daher auch in US-Patent 5 254 751 eine homogene CHP-Spaltung beschrieben, das heißt, die in nur geringen Mengen eingesetzte Schwefelsäure löst sich in der Reaktionsmasse. Im Gegensatz zu US-Patent 4 358 618 wird nach US-Patent 5 254 751 aber die CHP-Spaltung in drei hintereinander geschalteten Kühlern bei 45 bis 75 °C durchgerührt, wobei es sich offensichtlich um Rohrbündelwärmeübertrager handelt, in denen die Reaktionsmasse durch den Mantelraum unter Rohrströmungsbedingungen fließt. Das Reaktionsprodukt wird dabei teilweise zur Erzielung hoher Selektivitäten derart im Kreis gefahren, daß ein Teil des hinter dem letzten Kühler anfallenden CHP-Spaltproduktes zurückgeführt und dem zum ersten Kühler zuströmenden CHP-Massenstrom beigemischt wird, wobei das Verhältnis von zurückgeführtem Kreislaufmassenstrom zum zuströmenden CHP-Massenstrom, das sogenannte Kreislaufverhältnis λ, im Bereich 10 bis 25 liegen soll. Des weiteren wird beansprucht, daß im Ablauf aus dem Reaktorsystem noch 0,3 bis 1,5 Gew.-% Restgehalte an CHP vorliegen müssen. Das Spaltprodukt wird anschließend durch Erwärmung auf 80 bis 110 °C unter Rohrströmungsbedingungen behandelt, um das in der CHP-Spaltung gebildete DCP in Phenol, Aceton und AMS zu überführen.

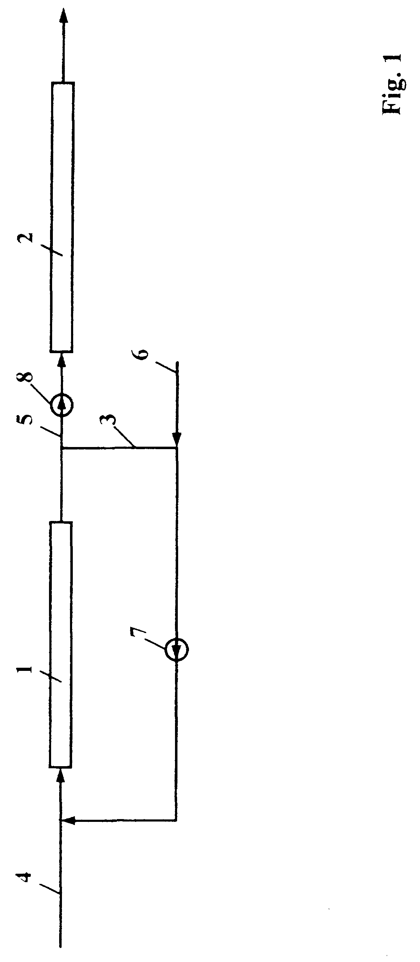

Fig. 1 zeigt ein Prinzipschema dieser in US-Patent 5 254 751 beschriebenen CHPund DCP-Spaltung. Im Reaktor 1 erfolgt unter Rohrströmungsbedingungen die CHP-Spaltung. Ein Teil des Spaltproduktes wird über Rohrleitung 3 abgezweigt und nach Zugabe von Säure als Katalysator über Leitung 6 mit dem Zustrom an technischem CHP über Leitung 4 vermischt, bevor dieses Gemisch in den Reaktor 1 eintritt Der andere Teil des Spaltproduktes wird über Rohrleitung 5 dem Rohrreaktor 2 zur DCP-Spaltung zugeführt. Das Kreislaufverhältnis λ ergibt sich als das Verhältnis von Kreislaufmassenstrom in Leitung 3 vor der CHP-Zumischung zum CHP-Zustrom über Leitung 4. Der Spaltproduktkreislauf wird über eine Pumpe 7 (Kreislaufpumpe) erzeugt. Das Spaltprodukt, das z. B. frei aus dem Kreislauf abläuft, wird mittels einer weiteren Pumpe 8 zum nachgeschalteten DCP-Reaktor 2 gefördert.

Durch die Verwendung von Reaktoren mit Rohrströmungscharakteristik ergibt sich zwar prinzipiell, wie bereits beschrieben, der Vorteil höherer Raum-Zeit-Ausbeuten und damit kleinerer Reaktionsvolumina, durch die geförderten hohen Kreislaufströme steigt das Volumen dieser Apparate aber wieder an, und es sind darüber hinaus große Rohrleitungen und Pumpen zur Realisierung dieser hohen Kreislaufmengen erforderlich.

Damit stellt sich die Aufgabe, ein verbessertes Verfahren zur säurekatalysierten homogenen Spaltung von CHP in Phenol und Aceton bereitzustellen, das neben einer hohen Selektivität eine Reduzierung der Investitionskosten aufweist.

Diese Aufgabe wird erfindungsgemäß gelöst gemäß Patentanspruch 1 durch ein Verfahren zur Herstellung von Phenol und Aceton durch säurekatalysierte homogene Spaltung von CHP in einer Spaltvorrichtung mit einem oder mehreren Reaktoren mit Rohrströmungscharakteristik für die CHP-Spaltung, wobei ein Teil des aus diesen Reaktoren hervorgehenden Spaltproduktstroms zurückgeführt und mit dem CHP-haltigen Zustrom zur Spaltvorrichtung vereint wird, das dadurch gekennzeichnet ist, daß das Massenstromverhältnis von zurückgeführtem Spaltproduktteilstrom zum CHP-haltigen Zustrom zur Spaltung kleiner als 10 ist.

Überraschenderweise wurde gefunden, daß entgegen der Lehre aus US-Patent 5 254 751 kein Kreislaufverhältnis λ von 10 bis 25 erforderlich ist, um hohe Selektivitäten bei der CHP-Spaltung zu erzielen. Es wurde vielmehr gefunden, daß durch eine Absenkung des Kreislaufverhältnisses unter 10, vorzugsweise auf 2 bis 9, besonders bevorzugt auf 5 bis 7, bei sonst gleichen Bedingungen sogar verbesserte Selektivitäten erzielt werden können.

Eigene Untersuchungen im Rahmen der vorliegenden Erfindung haben gezeigt, daß bei ansonsten gleichen Reaktionsbedingungen in einer vorgegebenen CHP-Spaltvorrichtung bei einer Vergrößerung des Kreislaufverhältnisses auch die Zugabe von Säure als Katalysator erhöht werden muß, um unverändert bei gleicher Verweilzeit der Reaktionsprodukte im Reaktorsystem eine CHP-Spaltung bis zu der gleichen vorgegebenen Restkonzentration im Spaltprodukt zu erzielen. Das stärker sauere Milieu während der Spaltreaktion begünstigt aber offensichtlich die Nebenproduktbildung und verschlechtert damit die Selektivität. Daher sind erfindungsgemäß stets möglichst kleine Kreislaufverhältnisse anzustreben.

Wie bereits erwähnt, ist es aus Sicherheitsgründen sicherzustellen, daß die bei der stark exothermen CHP-Spaltreaktion freiwerdende Reaktionswärme zu jeder Zeit und an jeder Stelle zur Vermeidung unkontrollierbarer Reaktorzustände sicher abgeführt werden kann, also auch, wenn im Störungsfall der Durchfluß durch die Spaltvorrichtung zum Erliegen kommt. Um redundante Ausführungen technischer Komponenten mit entsprechenden Uberwachungseinheiten zu vermeiden, wird in der Praxis bevorzugt, hinreichend große Wärmeübertragungsflächen bereitzustellen, die auch bei Störungen sicher für eine ausreichend hohe Wärmeabfuhr sorgen. Da mit sinkendem Kreislaufverhältnis die Eintrittskonzentration an CHP in die CHP-Spaltvorrichtung steigt (denn durch geringe Kreislaufströme wird das technische CHP weniger stark verdünnt), werden aus sicherheitstechnischen Gründen damit auch entsprechend größere Wärmeübertragungsflächen insbesondere an den Orten hoher CHP-Konzentration, also vor allem am Eintritt in die Spaltvorrichtung, erforderlich. Die Absenkung des Kreislaufverhältnisses ist also gewohnlich für eine vorgegebene Spaltvorrichtung aus sicherheitstechnischen Erwägungen nach unten begrenzt. Dies führt bei herkömmlichen CHP-Spaltvorrichtungen wie z. B. den bevorzugt eingesetzten Rohrbundelwarmeubertragern dazu, daß erfindungsgemäß besonders bevorzugt Kreislaufverhaltnisse von 5 bis 7 eingestellt werden, da diese noch leicht sicherheitstechnisch zu beherrschen sind. Durch entsprechende Dimensionierung der Apparate kann dabei z. B. sichergestellt werden, daß selbst bei Ausfall der Durchströmung der Reaktoren die Reaktionswärme durch freie Konvektion in der Reaktionsmasse oder andere Maßnahmen an die Umgebung abgeführt werden kann.

Hierzu kann z. B. der erste Reaktor als Rohrbündelapparat senkrecht aufgestellt werden, wobei die Reaktionsmasse von unten nach oben in den Rohren durch den Apparat strömt und das technische CHP unten zudosiert wird. In diesem Fall kann bei Ausfall des Kreislaufstromes die freiwerdende Wärme durch Ausbildung von freier Konvektion noch ausreichend abgeführt werden. Eine andere Möglichkeit besteht darin, die Pumpe für den Kreislaufstrom redundant auszuführen, wobei z. B zwei Kreiselpumpen parallel angeordnet sind und gleichzeitig laufen sollten. Bei Ausfall einer Pumpe wird der Kreislauf durch die zweite Pumpe noch ausreichend aufrecht erhalten. Weiterhin kann bei Ausfall des Kreislaufstromes mit ausreichend Cumol gespült und so die Reaktionsmasse gekühlt und verdünnt werden. All dies sind Maßnahmen, die nach dem heutigen Stand der Technik durchführbar sind, sie können natürlich auch kombiniert angewendet werden, um auch kleine Kreislaufverhältnisse sicher zu beherrschen.

Selbst kleinere Kreislaufverhältnisse unter 5 können so in Reaktoren, die eine volumenspezifische Oberfläche zur Wärmeübertragung (d. h. ein Verhältnis von Wärmeübertragungsoberfläche zu eingeschlossenem Reaktionsvolumen) von größer oder gleich 500 m²/m³ aufweisen, realisiert werden. Auch hier wird der Reaktor bevorzugt so bemessen, daß bei einem Ausfall der Strömung ein für das eingeschlossene Reaktionsvolumen ausreichend hoher Wärmedurchgangskoeffizient vorliegt, um eine sichere Wärmeabführ zu gewährleisten.

Zusätzlich zu diesem Vorteil der höheren Selektivität und damit der Verringerung des Rückstandes ergeben sich aber auch technische Vorteile durch die erfindungsgemäße Fahrweise mit kleinen Kreislaufverhältnissen Bei z. B. einem Kreislaufverhältnis von 7 ergibt sich für eine Anlage zur Erzeugung von 200.000 t/a Phenol ein Kreislaufvolumenstrom von 530 m³/h, während für ein Kreislaufverhältnis von 17 ein Kreislaufvolumenstrom von 1.200 m³/h einzustellen ist. Geht man weiter von technisch üblichen Reaktionszeiten von ca. einer Minute für die CHP-Spaltung aus, so ist bei einem Kreislaufverhältnis von 7 ein Reaktionsvolumen von ca. 9 m³ erforderlich, bei einem Kreislaufverhältnis von 17 wird dagegen ein Reaktionsvolumen von ca. 20 m³ benötigt. Somit sind die Baugröße und damit die Investitionskosten für Spaltanlagen mit kleineren Kreislaufverhältnissen deutlich geringer als diejenigen für Spaltanlagen mit hohen Kreislaufverhältnissen.

Ein weiterer Vorteil des erfindungsgemäßen Verfahrens besteht darin, daß durch Absenkung der Kreislaufverhältnisse auch niedrigere CHP-Restkonzentrationen im Spaltprodukt eingestellt werden können, ohne daß dadurch zwangsläufig die Nebenproduktbildung und damit die Selektivität verschlechtert wird. Zwar muß zur Verringerung der CHP-Restkonzentration der Säurezusatz erhöht werden, wodurch die Nebenproduktbildung begünstigt wird; dieser Effekt kann aber durch die Selektivitätssteigerung durch Absenken des Kreislaufverhältnisses zumindest kompensiert oder noch übertroffen werden, so daß zumindest ohne Einbußen bei der Selektivität niedrigere CHP-Restkonzentrationen erzielt werden können. Bei einer Fahrweise mit einer CHP-Restkonzentration von z. B. 1 Gew.-% oder mehr ist die Gefahr des "Durchschlagens" von CHP in nachgeschaltete Behälter größer als bei Fahrweisen mit kleinen Restkonzentrationen. Entsprechend muß der sicherheitstechnische Aufwand bei Anlagen mit hoher CHP-Restkonzentration im Spaltprodukt höher sein. Daher sind niedrige Restkonzentrationen stets anzustreben.

Mit Hilfe des erfindungsgemäßen Verfahrens wird vorzugsweise ein technisches CHP gespalten, das 65 bis 90 Gew.-% CHP enthalt. Der Rest besteht im wesentlichen aus Cumol; ferner sind in geringem Umfang bei der Oxidation von Cumol gebildete Nebenprodukte enthalten.

Die Auswahl der Reaktoren erfolgt in Abhängigkeit vom CHP-Gehalt des zu spaltenden technischen CHP unter Berücksichtigung der sicherheitstechnischen Aspekte derart, daß ein möglichst kleines Kreislaufverhältnis realisiert werden kann. Üblicherweise umfaßt eine CHP-Spaltvorrichtung erfindungsgemäß einen oder mehrere in Reihe geschaltete Reaktoren mit Rohrströmungscharakteristik, vorzugsweise Rohrbündelwärmeübertrager, wobei das Reaktionsgemisch entweder durch den Rohrraum - was bevorzugt ist - oder durch den Mantelraum strömt. Erfindungsgemäß geeignete Reaktoren sind darüber hinaus Platten- und Spiralwärmeübertrager, die ebenfalls eine Rohrströmungscharakteristik aufweisen. Insbesondere, aber nicht ausschließlich, für kleine Kreislaufverhältnisse werden bevorzugt Reaktoren mit Rohrströmungscharakteristik und einer volumenspezifischen Oberfläche zur Wärmeübertragung von 500 m²/m³ verwendet.

Nach dem letzten CHP-Spaltreaktor wird ein Teil des Spaltproduktstroms abgetrennt. Dieser abgetrennte Spaltproduktteilstrom wird zurückgeführt und vor oder im - bezogen auf die Durchströmung - ersten Spaltreaktor mit dem Zustrom an zu spaltendem technischen CHP vereint. Vorzugsweise erfolgt die Beimischung vor Eintritt in den ersten Reaktor. Die als Katalysator benötigte Säure wird vorzugsweise dem zurückgeführten Spaltproduktteilstrom zugesetzt, bevor dieser mit dem Zustrom an frischem technischen CHP vereint wird. Vorzugsweise wird als Katalysator Schwefelsäure verwendet. Bevorzugt wird soviel Säure zugesetzt und in dem Reaktionsgemisch gelöst, daß die Restkonzentration an CHP im Spaltprodukt von 0,1 bis 1,5 Gew.-%, vorzugsweise von 0,1 bis 0,3 Gew.-%, beträgt. Üblicherweise ist hierzu eine Säurekonzentration von 50 bis 500 Gew.-ppm im Reaktionsgemisch erforderlich. Die Spaltreaktion erfolgt erfindungsgemäß bevorzugt in einer Spaltvorrichtung mit drei bis sechs hintereinander angeordneten gekühlten Rohrbündelwärmeübertragern. Das mit rückgeführtem Spaltprodukt verdünnte technische CHP strömt entweder durch den Rohr- oder durch den Mantelraum. Bei Strömung durch den Mantelraum kann durch Einbau von Umlenkblechen oder Längsblechen der Kolbenströmungscharakter verbessert werden. Bei Strömung im Rohrraum können auch mehrgängige Apparate verwendet werden.

Die homogene Spaltung wird vorzugsweise bei 45 °C bis 75 °C und Reaktionsdrücken von 1 bis 5 bar absolut durchgeführt. Es kann ferner vorteilhaft sein, zusätzlich zur Säurezugabe dem Reaktionsgemisch in geringem Umfang Wasser zuzugeben, vorzugsweise sind 0,3 bis 1 Gew.-% Wasser im Reaktionsgemisch einzustellen. Bevorzugt erfolgt die Wasserzugabe ebenfalls bereits zum zurückgeführten Spaltproduktteilstrom, ggf. gemeinsam mit der Säure. Die Kühlung der Rohrbündelwärmeübertrager zur Abführ der Reaktionswärme erfolgt vorzugsweise mit Wasser. Die Verweilzeiten des Reaktionsgemisches in der CHP-Spaltvorrichtung betragen in der Regel 0,5 bis 10 Minuten.

Bei entsprechender Auslegung der herkömmlichen Apparate lassen sich erfindungsgemäß ohne sicherheitstechnische Bedenken Kreislaufverhältnisse von zurückgeführtem Spaltproduktteilstrom zu Zustrom an technischem CHP von vorzugsweise 5 bis 7 einstellen. Neben den Einsparungen bei den Apparatekosten und Betriebskosten können so Verbesserungen in der Cumolausbeute um ca. 0,5 Prozentpunkte oder mehr erreicht werden, die vor dem Hintergrund einer Jahresweltproduktion an Phenol von ca. 7 Mio. Tonnen eine signifikante Verbesserung der Wirtschaftlichkeit bedeuten.

Der nicht zurückgeführte Teil des CHP-Spaltproduktes wird wie z. B. in US 5 254 751 vorzugsweise einer geeigneten Spaltvorrichtung zur DCP-Spaltung, vorzugsweise zumindest einem Rohrreaktor, zugeführt, in der auf bekannte Weise vor der weiteren Aufbereitung des Stoffstroms zur Gewinnung von Phenol und Aceton eine DCP-Spaltung erfolgt.

Das erfindungsgemäße Verfahren wird durch die nachfolgenden Beispiele näher erläutert, ohne darauf beschränkt zu sein:

### Beispiel 1 (Vergleichsbeispiel):

In einer Spaltapparatur mit prinzipiellem Aufbau gemäß Fig. 1 wird technisches CHP mit einem CHP-Gehalt von 67 Gew.-% bis auf einen Restgehalt im CHP-Spaltprodukt von 1,0 Gew.-% umgesetzt. Dabei wird ein Kreislaufverhältnis λ von 17 eingestellt. Die Zugabe von Schwefelsäure als Katalysator ist entsprechend angepaßt.

Als CHP-Spaltreaktoren dienen drei in Reihe geschaltete Rohrbündelwärmeübertrager (Reaktor 1 in Fig. 1); die Reaktionstemperatur beträgt 50 °C. Der Reaktionsdruck beträgt 1 bar absolut.

Das gewonnene CHP-Spaltprodukt enthält 0,21 Gew.-% Hochsieder (im wesentlichen polymerisiertes AMS und Cumylphenole). An die CHP-Spaltung schließt sich die DCP-Spaltung an

### Beispiel 2 (erfindungsgemäß)

Die CHP-Spaltung aus Beispiel 1 wird bei ansonsten gleichen Bedingungen mit einem Kreislaufverhältnis von 7 betrieben; durch Anpassung der Säurezugabe wird eine CHP-Restkonzentration von 0,25 Gew.-% im CHP-Spaltprodukt eingestellt. Das Spaltprodukt enthält ebenfalls 0,21 Gew.-% Hochsieder.

Trotz der verringerten CHP-Restkonzentration, die in Beispiel 1 nur durch eine höhere Säurezugabe, die die Nebenproduktbildung begünstigt, erreicht werden kann, wird hier durch Absenkung des Kreislaufverhältnisses dieselbe Selektivität wie im Vergleichsbeispiel 1 erreicht Spaltreaktoren und Rohrleitungen könnten entsprechend kleiner dimensioniert sein Zudem bietet der geringere CHP-Restgehalt sicherheitstechnische Vorteile für die nachfolgenden Anlagenteile.

### Beispiel 3 (erfindungsgemaß):

Die CHP-Spaltung aus Beispiel 1 wird nun bei ansonsten gleichen Bedingungen einschließlich der CHP-Restkonzentration von 1,0 Gew.-% im Spaltprodukt mit einem Kreislaufverhältnis von 7 betrieben. Der Hochsiederanteil im Spaltprodukt beträgt 0,16 Gew.-%. Durch Absenkung des Kreislaufverhältnisses wurde die Selektivität gegenüber Beispiel 1 deutlich erhöht. Beim Vergleich mit Beispiel 2 zeigt sich, daß durch Anhebung der CHP-Restkonzentration bei niedrigem Kreislaufverhältnis die Selektivität verbessert werden kann.

### Beispiele 4-8 (erfindungsgemäß):

Die CHP-Spaltung aus Beispiel 1 wird bei ansonsten gleichen Bedingungen bei verschiedenen Kreislaufverhältnissen derart betrieben, daß bei angepaßter Säurezugabe sich stets eine CHP-Restkonzentration von 0,25 Gew.-% im Spaltprodukt einstellt. Ferner wird jeweils der Gehalt an Hochsiedern im Spaltprodukt in Abhängigkeit vom Kreislaufverhältnis bestimmt.

Die Ergebnisse zeigt Tabelle 1.

**Tabelle 1**

| Konzentration an Hochsiedern bei verschiedenen Kreislaufverhältnissen bei gleichbleibender CHP-Restkonzentration | | | | | |
|---|---|---|---|---|---|
| **Beispiel** | **4** | **5** | **6** | **7** | **8** |
| Kreislaufverhältnis | 9 | 8 | 7 | 6 | 5 |
| Konzentration an Hochsiedern (%) | 0,25 | 0,23 | 0,21 | 0,19 | 0,17 |

Hier wird deutlich, daß bei gleichbleibender CHP-Restkonzentration, aber geringerem Kreislaufverhältnis die Hochsiederbildung abnimmt, bzw. die Selektivität also zunimmt.

### Beispiel 9:

In einer Spaltapparatur mit prinzipiellem Aufbau gemäß US-Patent 4 358 618 wird technisches CHP mit einem CHP-Gehalt von 67 Gew.-% zunächst in einem ideal durchmischten Apparat bei 50 °C bis auf einen Restgehalt an CHP von 2,75 Gew.-% und anschließend in einem Rohrreaktor bei 50 °C bis zu einer CHP-Restkonzentration von 0,2 Gew.-% gespalten. Das Spaltprodukt enthält 0,20 Gew.-% Hochsieder.

Wie die Beispiele 4 bis 8 zeigen, sind erfindungsgemäß höhere Selektivitäten erreichbar.

## Patentansprüche

1. Verfahren zur Herstellung von Phenol und Aceton durch säurekatalysierte homogene Spaltung von Cumolhydroperoxid in einer Spaltvorrichtung mit einem oder mehreren Reaktoren mit Rohrströmungscharakteristik für die Cumolhydroperoxid-Spaltung, wobei ein Teil des aus diesen Reaktoren hervorgehenden Spaltproduktstroms zurückgeführt und mit dem Cumolhydroperoxid-haltigen Zustrom zur Spaltvorrichtung vereint wird,
**dadurch gekennzeichnet,**
**daß** das Massenstromverhältnis von zurückgeführtem Spaltproduktteilstrom zum Cumolhydroperoxid-haltigen Zustrom zur Spaltung kleiner als 10 ist.

2. Verfahren nach Anspruch 1,
**dadurch gekennzeichnet,**
**daß** die Reaktoren eine volumenspezifische Oberfläche zur Wärmeübertragung von größer oder gleich 500 m²/m³ aufweisen.

3. Verfahren nach zumindest einem der vorherigen Ansprüche,
**dadurch gekennzeichnet,**
**daß** die Spaltvorrichtung einen oder mehrere in Reihe geschaltete Rohrbündelwärmeübertrager als Reaktoren für die Cumolhydroperoxid-Spaltung umfaßt.

4. Verfahren nach Anspruch 3,
**dadurch gekennzeichnet,**
**daß** die Spaltvorrichtung drei bis sechs Rohrbündelwärmeübertrager als Reaktoren für die Cumolhydroperoxid-Spaltung aufweist.

5. Verfahren nach Anspruch 3 oder 4,
**dadurch gekennzeichnet,**
**daß** das Reaktionsgemisch durch den Rohrraum der Rohrbündelwärmeübertrager strömt.

6. Verfahren nach zumindest einem der vorherigen Ansprüche,
**dadurch gekennzeichnet,**
**daß** das Massenstromverhältnis von zurückgeführtem Spaltproduktteilstrom zum Cumolhydroperoxid-haltigen Zustrom im Bereich von 5 bis 7 liegt.

7. Verfahren nach zumindest einem der vorherigen Ansprüche,
**dadurch gekennzeichnet,**
**daß** die Restkonzentration an Cumolhydroperoxid im Cumolhydroperoxid-Spaltprodukt 0,1 bis 1,5 Gew.-% beträgt.

8. Verfahren nach Anspruch 7,
**dadurch gekennzeichnet,**
**daß** die Restkonzentration an Cumolhydroperoxid im Cumolhydroperoxid-Spaltprodukt 0,1 bis 0,3 Gew.-% beträgt.

9. Verfahren nach zumindest einem der vorherigen Ansprüche,
**dadurch gekennzeichnet,**
**daß** die Spaltung von Cumolhydroperoxid bei 45 °C bis 75 °C und Drücken von 1 bis 5 bar absolut erfolgt.

10. Verfahren nach zumindest einem der vorherigen Ansprüche,
**dadurch gekennzeichnet,**
**daß** die Verweilzeit des Reaktionsgemisches in der Cumolhydroperoxid-Spaltvorrichtung 0,5 bis 10 Minuten beträgt.

11. Verfahren nach zumindest einem der vorherigen Ansprüche,
**dadurch gekennzeichnet,**
**daß** der Cumolhydroperoxidgehalt im Zustrom 65 bis 90 Gew.-% beträgt.

12. Verfahren nach zumindest einem der vorherigen Ansprüche,
**dadurch gekennzeichnet,**
**daß** als Säurekatalysator Schwefelsäure dient.

## Claims

1. Process for preparing phenol and acetone by acid-catalyzed homogeneous cleavage of cumene hydroperoxide in a cleavage apparatus having one or more reactors with tube flow characteristics for the cumene hydroperoxide cleavage, whereby a portion of the cleavage product stream obtained from these reactors is recycled and combined with the cumene hydroperoxide-containing feed stream fed to the cleavage apparatus, **characterized in that** the mass flow ratio of the recycled portion of the cleavage product stream to the cumene hydroperoxide-containing feed stream fed to the cleavage is less than 10.

2. Process according to claim 1, **characterized in that** the reactors have a specific surface area for heat transfer per unit volume of **≥** 500 m²/m³.

3. Process according to any of the preceding claims, **characterized in that** the cleavage apparatus comprises one or more tube bundle heat exchangers connected in series as reactors for cleavage of cumene hydroperoxide.

4. Process according to claim 3, **characterized in that** the cleavage apparatus has from three to six tube bundle heat exchangers as reactors for the cleavage of cumene hydroperoxide.

5. Process according to claim 3 or 4, **characterized in that** the reaction mixture flows through the tubular space of the tube bundle heat exchangers.

6. Process according to at least one of the preceding claims, **characterized in that** the mass flow ratio of the recycled portion of the cleavage product stream to the cumene hydroperoxide-containing feed stream is in the range of 5 to 7.

7. Process according to at least one of the preceding claims, **characterized in that** the residual concentration of cumene hydroperoxide in the cumene hydroperoxide cleavage product ranges from 0.1 to 1.5 percent by weight.

8. Process according to claim 7, **characterized in that** the residual concentration of cumene hydroperoxide in the cumene hydroperoxide cleavage product ranges from 0.1 to 0.3 percent by weight.

9. Process according to at least one of the preceding claims, **characterized in that** the cleavage of cumene hydroperoxide is conducted at 45°C to 75°C and at a pressure of 1 to 5 bar absolute.

10. Process according to at least one of the preceding claims, **characterized in that** the residence time of the reaction mixture in the cumene hydroperoxide cleavage apparatus ranges from 0.5 to 10 minutes.

11. Process according to at least one of the preceding claims, **characterized in that** the content of the cumene hydroperoxide in the feed stream ranges from 65 to 90 percent by weight.

12. Process according to at least one of the preceding claims, **characterized in that** the acid catalyst is sulfuric acid.

## Revendications

1. Procédé de préparation de phénol et d'acétone par décomposition homogène catalysée par un acide d'hydroperoxyde de cumène dans un dispositif de décomposition comprenant un ou plusieurs réacteurs à caractéristique d'écoulement tubulaire pour la décomposition d'hydroperoxyde de cumène, où une partie du courant de produit de décomposition provenant de ces réacteurs est recyclée et réunie au courant d'alimentation contenant de l'hydroperoxyde de cumène vers le dispositif de décomposition, **caractérisé en ce que** le rapport de débit massique du courant partiel de produit de décomposition recyclé au courant d'alimentation contenant de l'hydroperoxyde de cumène vers la décomposition est inférieur à 10.

2. Procédé selon la revendication 1, **caractérisé en ce que** les réacteurs présentent une surface spécifique volumique de transfert de chaleur supérieure ou égale à 500 m²/m³.

3. Procédé selon l'une au moins des revendications qui précèdent, **caractérisé en ce que** le dispositif de décomposition comprend un ou plusieurs échangeurs de chaleur à faisceau de tubes raccordés en série en tant que réacteurs pour la décomposition de l'hydroperoxyde de cumène.

4. Procédé selon la revendication 3, **caractérisé en ce que** le dispositif de décomposition présente de trois à six échangeurs de chaleur à faisceau de tubes en tant que réacteurs pour la décomposition de l'hydroperoxyde de cumène.

5. Procédé selon la revendication 3 ou 4, **caractérisé en ce que** le mélange réactionnel s'écoule dans l'espace tubulaire de l'échangeur de chaleur à faisceau de tubes.

6. Procédé selon l'une au moins des revendications qui précèdent, **caractérisé en ce que** le rapport de débit massique du courant partiel de produit de décomposition recyclé au courant d'alimentation contenant de l'hydroperoxyde de cumène est de l'ordre de 5 à 7.

7. Procédé selon l'une au moins des revendications qui précèdent, **caractérisé en ce que** la concentration résiduelle d'hydroperoxyde de cumène dans le produit de décomposition d'hydroperoxyde de cumène est de 0,1 à 1,5% en poids.

8. Procédé selon la revendication 8, **caractérisé en ce que** la concentration résiduelle d'hydroperoxyde de cumène dans le produit de décomposition d'hydroperoxyde de cumène est de 0,1 à 0,3% en poids.

9. Procédé selon l'une au moins des revendications qui précèdent, **caractérisé en ce que** la décomposition de l'hydroperoxyde de cumène est entreprise à des températures de 45°C à 75°C et des pressions de 1 à 5 bar absolus.

10. Procédé selon l'une au moins des revendications qui précèdent, **caractérisé en ce que** le temps de séjour du mélange réactionnel dans le dispositif de décomposition d'hydroperoxyde de cumène est de 0,5 à 10 minutes.

11. Procédé selon l'une au moins des revendications qui précèdent, **caractérisé en ce que** la teneur en hydroperoxyde de cumène dans le courant d'alimentation est de 65 à 90% en poids.

12. Procédé selon l'une au moins des revendications qui précèdent, **caractérisé en ce que** le catalyseur acide utilisé est de l'acide sulfurique.
